# EUROPÄISCHE PATENTSCHRIFT

(11) **EP 3 194 067 B1**
(45) Veröffentlichungstag und Bekanntmachung des Hinweises auf die Patenterteilung: **11.07.2018**
(21) Anmeldenummer: 15759658.6
(22) Anmeldetag: 17.08.2015
(51) Int. Cl.: B01J 19/24, C01B 32/80, C07C 68/02, B01J 19/00

(54) **VERFAHREN ZUR PHOSGENIERUNG VON HYDROXYL-, THIOL-, AMINO- UND/ODER FORMAMIDGRUPPEN ENTHALTENDEN VERBINDUNGEN**
METHOD FOR PHOSGENATING COMPOUNDS CONTAINING HYDROXYL, THIOL, AMINO AND/OR FORMAMIDE GROUPS
PROCÉDÉ DE PHOSGÉNATION DE COMPOSÉS CONTENANT DES GROUPES HYDROXYLE, THIOL, AMINO ET/OU FORMAMIDE

(30) Priorität: 20.08.2014 DE 102014111902
(43) Veröffentlichungstag der Anmeldung: 26.07.2017
(73) Patentinhaber: Bayer Aktiengesellschaft, 51373 Leverkusen (DE)
(72) Erfinder: MLECZKO, Leslaw, 41542 Dormagen (DE); WOLF, Aurel, 42489 Wülfrath (DE); SCHELLEN,Ralph, 41541 Dormagen (DE); METAXAS, Konstantinos, 50670 Köln (DE); ROGGAN, Jens Stefan, 50679 Köln (DE)
(74) Vertreter: BIP Patents
(86) Internationale Anmeldenummer: PCT/EP2015/068811
(87) Internationale Veröffentlichungsnummer: WO 2016/026799

(56) Entgegenhaltungen:
- EP-A1- 0 520 238
- WO-A1-2006/089429

## Beschreibung

Die Arbeiten, die zu dieser Erfindung geführt haben, wurden gemäß der Finanzhilfevereinbarung Nr. 245988-1 im Zuge des Siebten Rahmenprogramms der Europäischen Union (FP7/2007-2013)-INCAS (Integration of Nanoreactor and multisite Catalysis for a Sustainable chemical production) gefördert.

Die vorliegende Erfindung betrifft ein Verfahren zur Reaktion von Phosgen mit Hydroxyl-, Thiol-, Amino- und/oder Formamidgruppen enthaltenden Verbindungen, umfassend die Schritte: (I) Bereitstellen eines Reaktors, welcher einen ersten Reaktionsraum und einen zweiten Reaktionsraum umfasst, wobei der erste und der zweite Reaktionsraum durch eine poröse Kohlenstoff-Membran voneinander getrennt sind; (II) Bereitstellen von Kohlenmonoxid und Chlor in dem ersten Reaktionsraum; sowie gleichzeitig (III) Bereitstellen einer Hydroxyl-, Thiol-, Amino- und/oder Formamidgruppen enthaltenden Verbindung in dem zweiten Reaktionsraum. Sie betrifft weiterhin einen Reaktor, der zur Durchführung des erfindungsgemäßen Verfahrens geeignet ist.

Phosgen (COCl₂) ist ein Schlüsselreagenz in der Herstellung von Pharmazeutika, Polyurethanen und Polycarbonaten. Es ist eine sehr reaktive aber auch sehr toxische Chemikalie, und der großtechnische Herstellungsprozess birgt aufgrund der in einer Anlage vorhandenen Phosgenmengen (hold-up) immer Risiken für die Umwelt im Falle einer unbeabsichtigten Freisetzung durch Lecks in Rohrleitungen oder anderen Schäden an Anlagenteilen.

Ein Beispiel für die großtechnische Verwendung von Phosgen als Schlüsselreagenz ist die Herstellung von Diphenylcarbonat (DPC). DPC ist ein wichtiges Zwischenprodukt für die Synthese qualitativ hochwertiger Polycarbonate, zum Beispiel durch Umesterung mit Bisphenol A. Die Synthese von DPC ausgehend von Phenol und Phosgen (Direktphosgenierung) verläuft in zwei Schritten: der erste Schritt umfasst die Herstellung von Phosgen in einer Gasphasenreaktion aus Kohlenmonoxid und Chlor, welche typischerweise über Aktivkohlekatalysatoren in einem Multiröhren-Festbettreaktor geschieht. Je nach Siedetemperatur des Kühlmediums in den Reaktoren unterscheidet man zwischen einer Phosgenherstellung in Kaltvereinigem oder Heissvereinigern. Durch Reaktion von Phenol mit Phosgen in Gegenwart eines geeigneten Katalysators erhält man schließlich DPC. Die DPC-Herstellung über Phenol-Direktphosgenierung bietet dabei im Vergleich zu den herkömmlichen Phasengrenzflächenverfahren (Reaktion von Natriumphenolat mit Phosgen) den Vorteil, dass die Bildung großer Mengen an NaCl Abfallprodukten vermieden wird. werden.

Sowohl die Phosgen- als auch die DPC-Synthese sind stark exotherme Reaktionen mit Reaktionsenthalpien von -107 und -54 kJ/mol. Insbesondere die Exothermie der Phosgensynthese in der Gasphase erfordert effiziente Kühlungssysteme, doch lassen sich sogenannten Hot Spots im Reaktor mit lokalen Temperaturen von über 500 °C nicht verhindern (vgl. Mitchell et al., Catal. Sci. Technol., 2012). Das Auftreten von Temperaturen von über 300 °C führt nicht nur zu einer erhöhten Materialbeanspruchung im Reaktor, sondern beeinflusst in negativer Weise die Gleichgewichtsreaktion der Phosgenbildung (der Zerfall des Phosgens überwiegt bei über 300 °C) und erhöht zudem die Desaktivierungsrate des Katalysators, so dass insgesamt die Raum-Zeit-Ausbeute und Prozess-Effizient sinken.

Unter dem Gesichtspunkt geringerer Hold-Up-Volumina zur Verbesserung der Prozess-Sicherheit sind mikrostrukturierte Reaktoren von Interesse. So beschreibt US 6,932,951 einen mikrostrukturierten Reaktor für die Hydrierung von Cyclohexen zu Cyclohexan als Beispielanwendung.

CN 101757859 A beschreibt einen Kohlenstoffmembranreaktor und ein Verfahren zur Verwendung desselben. Der Kohlenstoffmembranreaktor zeichnet sich dadurch aus, dass eine defektfreie Kohlenstoffmembran mit dem Gehäuse des Reaktors verbunden ist und ein Hohlraum innerhalb des Gehäuses des Reaktors gebildet wird, wobei der mit einer Beschickungsöffnung und einer Ablassöffnung für Reaktionspartner kommunizierende Hohlraum eine Beschickungsseite bildet und der Hohlraum mit einem Einlass und einem Auslass für Spülgas kommuniziert eine Durchlass-Seite bildet. Die defektfreie Kohlenstoffmembran ist mit Katalysatoren gefüllt, alternativ werden die Katalysatoren auf der defektfreien Kohlenstoffmembran vorgelegt.

Ein Übersichtsartikel zum Thema Kohlenstoffmembranen ist "A review on the latest development of carbon membranes for gas separation", A.F. Ismail, L.I.B. David/Journal of Membrane Science 193 (2001) 1-18. Eine weitere Publikation ist "Porous, catalytically active ceramic membranes for gas-liquid reactions: a comparison between catalytic diffuser and forced through flow concept", M. Reif, R. Dittmeyer, Catalysis Today 82 (2003) 3-14.

Betrachtet man die aktuelle Entwicklung so wird ein Bedarf an einem Verfahren mit dem reduzierten Phosgen-Holdup erkennbar. Im Rahmen der vorliegenden Erfindung wird ein solches Verfahren bereitgestellt. Insbesondere hat sich die Erfindung die Aufgabe gestellt, ein Phosgenierungsverfahren bereitzustellen, bei dem möglichst geringe Mengen an freiem Phosgen in der Reaktionsanlage vorliegen.

Erfindungsgemäß gelöst wird diese Aufgabe durch ein Verfahren zur Reaktion einer ersten Verbindung mit einer zweiten Verbindung,
wobei die erste Verbindung eine Gefahrstoffkennzeichnung gemäß GHS von GHS06 aufweist und aus der Reaktion von mindestens einer ersten fluiden Vorläuferverbindung und einer zweiten fluiden Vorläuferverbindung erhältlich ist
und wobei die zweite Verbindung mit der ersten Verbindung zu einer chemischen Reaktion in der Lage ist,
umfassend die Schritte:
(I) Bereitstellen eines Reaktors, welcher einen ersten Reaktionsraum und einen zweiten Reaktionsraum umfasst, wobei der erste und der zweite Reaktionsraum durch eine poröse Kohlenstoff-Membran voneinander getrennt sind;
(II) Bereitstellen der ersten und der zweiten Vorläuferverbindung im ersten Reaktionsraum; sowie gleichzeitig
(III) Bereitstellen der zweiten Verbindung in dem zweiten Reaktionsraum;
wobei die poröse Kohlenstoff-Membran eingerichtet ist, um:
- die Reaktion der ersten und zweiten Vorläuferverbindung zur ersten Verbindung zu katalysieren und
- die gebildete erste Verbindung in den zweiten Reaktionsraum übergehen zu lassen.

Es ist erfindungsgemäß vorgesehen, dass die erste Verbindung eine Gefahrstoffkennzeichnung gemäß GHS (Globally Harmonized System of Classification, Labelling and Packaging of Chemicals der Vereinten Nationen) von GHS06 aufweist. In der Europäischen Union existiert hierzu das Regelwerk der Verordnung (EG) Nr. 1272/2008, auch CLP-Verordnung genannt. Die Einstufung GHS06 weist auf giftige oder sehr giftige Stoffe hin.

Hinsichtlich der ersten fluiden Vorläuferverbindung und der zweiten fluiden Vorläuferverbindung sind erfindungsgemäß Gase und Flüssigkeiten, einschließlich Lösungen von Feststoffen in einem Lösungsmittel, vorgesehen.

Insbesondere kann die erste Verbindung Phosgen sein, die erste Vorläuferverbindung Kohlenmonoxid sein, die zweite Vorläuferverbindung Chlor sein und die zweite Verbindung eine Hydroxyl-, Thiol-, Amino- und/oder Formamidgruppen enthaltende Verbindung sein.

Aufgrund der herausragenden Bedeutung der Reaktion von Phosgen mit einer Hydroxyl-, Thiol-, Amino- und/oder Formamidgruppen enthaltenden Verbindung wird die vorliegende Erfindung im Zusammenhang mit dieser ersten und zweiten Verbindung erläutert, ohne hierauf beschränkt zu sein.

Im erfindungsgemäßen Verfahren tritt Phosgen nur als vergleichsweise kurzlebiges Intermediat auf. Das Gasgemisch aus Kohlenmonoxid und Chlor, welches im ersten Reaktionsraum vorliegt, reagiert bei Durchtritt durch die katalytisch aktive Kohlenstoff-Membran zu Phosgen. Das *in situ* gebildete Phosgen tritt aus den Poren der Kohlenstoff-Membran in den zweiten Reaktionsraum über, wo es mit der Hydroxyl-, Thiol-, Amino- und/oder Formamidgruppen enthaltenden Verbindung reagiert.

Durch das erfindungsgemäße Verfahren kann vermieden werden, dass in der Reaktionsanlage größere Mengen an Phosgen vorliegen. Ein weiterer Vorteil ist die Vermeidung von lokalen Hot Spots in der Phosgensynthese, wie sie von konventionellen Anlagen bekannt sind. Die Hydroxyl-, Thiol-, Amino- und/oder Formamidgruppen enthaltenden Verbindung dient ferner der Abfuhr der Reaktionswärme. Eine geringe Dicke der Membran unterstützt ebenfalls die Wärmeabfuhr. Zusätzlich wird die Bildung von NaCl als Nebenprodukt gegenüber dem konventionellen Phasentransfer-Verfahren vermieden. Insgesamt erhöht sich durch die Integration von zwei Reaktionen in einem Verfahren die Raum-Zeit-Ausbeute des Verfahrens über einen längeren Zeitraum und die thermische Belastung der Anlage wird verringert.

Gemäß Schritt (I) des erfindungsgemäßen Verfahrens wird ein Reaktor bereitgestellt. Die Bauform des Reaktors ist zunächst nicht weiter festgelegt und kann beispielsweise ein Rohrreaktor für den kontinuierlichen Betrieb oder ein Kesselreaktor für eine diskontinuierliche Fahrweise sein. Der Reaktor weist zwei Reaktionsräume auf, welche durch eine poröse Kohlenstoff-Membran voneinander getrennt sind. Ein Reaktionsraum ist für die Phosgenbildung und ein Reaktionsraum für die Phosgenierung vorgesehen. Durch die Wahl geeigneter Flüssigkeits- und Gasdrücke in den beiden Reaktionsräumen kann das Übertreten von flüssigen Reaktanden aus dem zweiten Reaktionsraum in den ersten Reaktionsraum unterbunden werden.

Die poröse Kohlenstoff-Membran ("Carbon-Membrane") kann eine frei tragende oder eine durch ein gasdurchlässiges Substrat geträgerte Membran sein. Sie kann durch Pyrolyse organischer Vorläuferverbindungen oder aber aus zuvor hergestelltem Kohlenstoffmaterial wie Aktivkohle, Graphen oder Kohlenstoffnanoröhrchen (CNT) erhalten werden. Sofern die Porosität der Membran für den Durchtritt von Phosgen geeignet ist, lassen sich unter dem Vorbehalt einer katalytischen Aktivität für die Phosgensynthese Kohlenstoff-Membranen aus dem Bereich der technischen Gastrennung einsetzen.

Der Begriff "porös" im Zusammenhang mit den Kohlenstoff-Membranen bedeutet hierbei, dass in der Membran miteinander verbundene Poren vorliegen, welche zumindest für die gebildeten Phosgenmoleküle einen Weg durch die Membran hindurch ermöglichen.

Die Schritte (II) und (III) des erfindungsgemäßen Verfahrens werden gleichzeitig durchgeführt, damit das *in situ* gebildete Phosgen möglichst schnell weiter reagieren kann. Beispiele für geeignete Hydroxyl-, Thiol-, Amino- und/oder Formamidgruppen enthaltenden Verbindungen sind aromatische Alkohole wie Phenol, aliphatische Alkohole, primäre aromatische Amine, sekundäre aromatische Amine, primäre aliphatische Amine, sekundäre aliphatische Amine, N,N-Dimethylformamid und N-Methylformanilid. Insbesondere aromatische und aliphatische Alkohole und Formamide sind bevorzugt; Erstere wegen der Anwendung der Reaktionsprodukte in der Polycarbonatherstellung und Letztere wegen ihres Einsatzes in Vilsmeyer-Haack-Formylierungen. Weiterhin bevorzugt sind primäre Amine, da sie durch Phosgenierung in die korrespondierenden Isocyanate überführt werden können, welche in der Polyurethanherstellung verwendet werden.

Insgesamt kann somit die Membran auch als Porenreaktor angesehen werden.

Gegen Korrosion empfindliche Oberflächen im Reaktor können zum Beispiel durch eine Edelstahl-oder SiO₂-Beschichtung geschützt werden.

Hinsichtlich der Reaktionsbedingungen im erfindungsgemäßen Verfahren kann die Reaktionstemperatur für die Phosgensynthese vorteilhafterweise zwischen 80 und 300 °C und für die Phosgenierung (insbesondere von Phenol) zwischen 150 und 300 °C liegen. Besonders bevorzugt ist eine Reaktionstemperatur im ersten und im zweiten Reaktionsraum von 190 bis 210 °C.

Der Druck im ersten und im zweiten Reaktionsraum kann beispielsweise 1 bis 29 bar betragen. Bevorzugt ist ein Druck von 24 bis 26 bar. Insbesondere im bevorzugten Bereich kann die Verweilzeit so reduziert werden, dass sie einige Minuten (im Gegensatz zu einer Stunde oder mehr) beträgt.

Es ist bei Phosgenierungsreaktionen zudem vorteilhaft, wenn die poröse Kohlenstoff-Membran ferner eingerichtet ist, um den Kontakt von Cl₂ mit den Ausgangsstoffen und Produkten im zweiten Reaktionsraum zu verhindern. Auf diese Weise lässt sich die Bildung von Chlorierungsprodukten wie beispielsweise Chlorphenolen verhindern.

Weitere Ausführungsformen und Aspekte der vorliegenden Erfindung werden nachfolgend erläutert. Sie können beliebig miteinander kombiniert werden, sofern sich aus dem Kontext nicht eindeutig das Gegenteil ergibt.

In einer Ausführungsform des erfindungsgemäßen Verfahrens weist die poröse Kohlenstoff-Membran eine nominale Porengröße, bestimmt mittels Quecksilber-Porosimetrie (ISO 15901-1), von ≥ 0,01 bis ≤ 10 µm auf. Die nominale Porengröße versteht sich wie üblich als das Maximum der Porengrößenverteilung. Bevorzugte nominale Porengrößen sind ≥ 0,1 bis ≤ 1,0 µm.

Die Membran weist vorzugsweise jeweils unabhängig voneinander die folgenden weiteren Eigenschaften auf:
Dicke: ≥ 1 bis ≤ 10 mm
Spezifische Oberfläche (BET): ≥ 100 bis ≤ 2000 m²/g
Porosität: ≥ 0,1 bis ≤ 0,5
Tortuosität: ≥ 1 bis ≤ 15
Wärmeleitfähigkeit: ≥ 1 bis ≤ 175 W/m/K
Membranbeladung im Reaktor: ≥ 300 bis ≤ 800 kg/m³

In einer weiteren Ausführungsform des erfindungsgemäßen Verfahrens umfasst die poröse Kohlenstoff-Membran weiterhin einen Katalysator für die Reaktion der ersten Verbindung (vorzugsweise von Phosgen) mit der zweiten Verbindung (vorzugsweise der Hydroxyl-, Thiol-, Amino- und/oder Formamidgruppen enthaltenden Verbindung), welcher zumindest teilweise auf der dem zweiten Reaktionsraum zugewandten Seite der porösen Kohlenstoff-Membran angeordnet ist. Zweckmäßigerweise handelt es sich um einen heterogenen Katalysator. Im Fall der Phosgenierung von aromatischen Alkoholen wie Phenol kann beispielsweise Al₂O₃ eingesetzt werden.

In einer weiteren Ausführungsform des erfindungsgemäßen Verfahrens liegt im zweiten Reaktionsraum weiterhin ein homogener Katalysator vor. Der Katalysator vorzugsweise für die Reaktion von Phosgen mit der Hydroxyl-, Thiol-, Amino- und/oder Formamidgruppen enthaltenden Verbindung ist somit in dem Reaktionsmedium, welches sich im zweiten Reaktionsraum befindet, gelöst. Im Fall der Phosgenierung von aromatischen Alkoholen wie Phenol können beispielsweise TiCl₄ oder Pyridin eingesetzt werden.

In einer weiteren Ausführungsform des erfindungsgemäßen Verfahrens liegt im ersten Reaktionsraum weiterhin ein offenzelliger Schaum vor. Grundsätzlich sind alle Schaum-Materialien geeignet, die unter den bei der Phosgensynthese herrschenden Temperaturen und insbesondere bis 300 °C stabil sind. Vorzugsweise handelt es sich um einen Metall- oder Keramikschaum. Neben einer besseren Durchmischung der Reaktanden CO und Cl₂ besitzt ein Schaum zudem die Eigenschaft, dass der erste Reaktionsraum durch ihn mechanisch abgestützt werden kann. Dieses ist insbesondere in mehrschichtigen Reaktoren von Vorteil.

In einer weiteren Ausführungsform des erfindungsgemäßen Verfahrens umfasst der Reaktor weiterhin einen Hohlraum zur Aufnahme eines Wärmeüberträgerfluids. Auf diese Weise können Wärmetauscher, insbesondere Kreuzstromwärmetauscher implementiert werden. Als Wärmeüberträgerfluid können sowohl Flüssigkeiten wie Wasser oder Öl als auch Gase wie Luft eingesetzt werden.

In einer weiteren Ausführungsform des erfindungsgemäßen Verfahrens umfasst der Reaktor weiterhin eine Verweilstrecke zur Vervollständigung der Reaktion der ersten Verbindung (vorzugsweise von Phosgen) mit der zweiten Verbindung (vorzugsweise mit der Hydroxyl-, Thiol-, Amino- und/oder Formamidgruppen enthaltenden Verbindung). Gerade bei mehrstufigen Reaktionen, in denen beispielsweise die Reaktion von Phenol mit Phosgen zum intermediär gebildeten Chloroformiat schnell, aber die weitere Reaktion des Chloroformiats mit Phenol zu DPC langsamer abläuft, kann durch eine Verweilstrecke im zweiten Reaktionsraum in Strömungsrichtung nach der Phosgensynthese (so dass kein zusätzliches Phosgen in den zweiten Reaktionsraum übergeht) die Ausbeute der Reaktion gesteigert werden.

In einer weiteren Ausführungsform des erfindungsgemäßen Verfahrens ist die Hydroxyl-, Thiol-, Amino- und/oder Formamidgruppen enthaltenden Verbindung Phenol, Dimethylformamid oder N-Methylformanilid.

In einer weiteren Ausführungsform des erfindungsgemäßen Verfahrens umfasst der Reaktor eine Mehrzahl von ersten Reaktionsräumen, zweiten Reaktionsräumen und porösen Kohlenstoff-Membranen, wobei jeweils ein erster und zweiter Reaktionsraum durch eine poröse Kohlenstoff-Membran voneinander getrennt sind. So können flache, mehrschichtige und modulare Membranreaktoren erhalten werden.

In einer weiteren Ausführungsform des erfindungsgemäßen Verfahrens ist der Reaktor zylindrisch aufgebaut mit von innen nach außen konzentrisch angeordnetem ersten Reaktionsraum und zweiten Reaktionsraum, wobei der erste und der zweite Reaktionsraum durch die poröse Kohlenstoff-Membran voneinander getrennt sind. Dann verhält sich der Reaktor im Prinzip wie ein Blasensäulenreaktor. Vorzugsweise sind mehrere dieser Reaktoren zu einem Rohrbündelreaktor zusammengefasst.

Der einzelne zylindrische Reaktor kann die folgenden Eigenschaften unabhängig voneinander aufweisen:
Durchmesser des zweiten Reaktionsraums: ≥ 3 bis ≤10 cm
Länge des zweiten Reaktionsraums: ≥ 3 bis ≤ 20 m
Verweilzeit der Reaktionsmischung im zweiten Reaktionsraum: ≥ 1 bis ≤ 60 Minuten
Molarer Überschuss an Phenol: ≥ 4 bis ≤ 6

In einer weiteren Ausführungsform des erfindungsgemäßen Verfahrens weisen der erste

Reaktionsraum und/oder der zweite Reaktionsraum eine Querschnittsfläche rechtwinklig zur Strömungsrichtung des durchströmenden Fluids von ≥ 8 · 10⁻⁵ bis ≤ 8 · 10⁻⁴ m² auf. Vorzugsweise beträgt die Querschnittsfläche ≥ 1 · 10⁻⁴ bis ≤ 7 · 10⁻⁴ m² und mehr bevorzugt ≥ 2 · 10⁻⁴ bis ≤ 6 · 10⁻⁴ m²·

In einer weiteren Ausführungsform des erfindungsgemäßen Verfahrens umfasst der Reaktor eine Mehrzahl von ersten Reaktionsräumen, die von einem gemeinsamen zweiten Reaktionsraum umgeben sind.

Neben der planaren Bauform ist eine Form der Kohlenstoff-Membran bevorzugt, in der sie als an einem Ende geschlossener Hohlzylinder ausgebildet ist.

Die Erfindung betrifft weiterhin einen Reaktor zur Reaktion von Phosgen mit Hydroxyl-, Thiol-, Amino- und/oder Formamidgruppen enthaltenden Verbindungen, umfassend:
- einen ersten Reaktionsraum und einen zweiten Reaktionsraum, wobei der erste und der zweite Reaktionsraum durch eine poröse Kohlenstoff-Membran voneinander getrennt sind;
   und
- einen Katalysator für die Reaktion von Phosgen mit der Hydroxyl-, Thiol-, Amino- und/oder Formamidgruppen enthaltenden Verbindung, welcher zumindest teilweise auf der dem zweiten Reaktionsraum zugewandten Seite der porösen Kohlenstoff-Membran angeordnet ist.

Zweckmäßigerweise handelt es sich um einen heterogenen Katalysator. Im Fall der Phosgenierung von aromatischen Alkoholen wie Phenol kann beispielsweise Al₂O₃ eingesetzt werden.

In einer Ausführungsform des erfindungsgemäßen Reaktors liegt im ersten Reaktionsraum weiterhin ein offenzelliger Schaum vor. Grundsätzlich sind alle Schaum-Materialien geeignet, die unter den bei der Phenolsynthese herrschenden Temperaturen und insbesondere bis 300 °C stabil sind. Vorzugsweise handelt es sich um einen Metall- oder Keramikschaum. Neben einer besseren Durchmischung der Reaktanden CO und Cl₂ hat ein Schaum die weitere Eigenschaft, dass der erste Reaktionsraum mechanisch abgestützt werden kann. Dieses ist insbesondere in mehrschichtigen Reaktoren von Vorteil.

In einer weiteren Ausführungsform des erfindungsgemäßen Reaktors weisen der erste Reaktionsraum und/oder der zweite Reaktionsraum eine Querschnittsfläche rechtwinklig zur Strömungsrichtung des durchströmenden Fluids von ≥ 8 · 10⁻⁵ bis ≤ 8 · 10⁻⁴ m² auf.

In einer weiteren Ausführungsform des erfindungsgemäßen Reaktors weist die poröse Kohlenstoff-Membran eine nominale Porengröße, bestimmt mittels Quecksilber-Porosimetrie (ISO 15901-1), von ≥ 0,01 bis ≤ 10 µm auf. Die nominale Porengröße versteht sich wie üblich als das Maximum der Porengrößenverteilung. Bevorzugte nominale Porengrößen sind ≥ 0,1 bis ≤ 1 µm.

Die Membran weist vorzugsweise jeweils unabhängig voneinander die folgenden weiteren Eigenschaften auf:
Dicke: ≥ 1 bis ≤ 10 mm
Spezifische Oberfläche (BET): ≥ 100 bis ≤ 2000 m²/g
Porosität: ≥ 0,1 bis ≤ 0,5
Tortuosität: ≥ 1 bis ≤ 15
Wärmeleitfähigkeit: ≥ 1 bis ≤ 175 W/m/K
Membranbeladung im Reaktor: ≥ 300 bis ≤ 800 kg/m³

In einer weiteren Ausführungsform des erfindungsgemäßen Reaktors umfasst der Reaktor weiterhin einen Hohlraum zur Aufnahme eines Wärmeüberträgerfluids. So können Wärmetauscher, insbesondere Kreuzstromwärmetauscher realisiert werden. Als Wärmeüberträgerfluid können sowohl Flüssigkeiten wie Wasser oder Öl als auch Gase wie Luft eingesetzt werden.

In einer weiteren Ausführungsform des erfindungsgemäßen Reaktors umfasst der Reaktor weiterhin eine Verweilstrecke zur Vervollständigung der Reaktion von Phosgen mit der Hydroxyl-, Thiol-, Amino- und/oder Formamidgruppen enthaltenden Verbindung. Gerade bei mehrstufigen Reaktionen, in denen beispielsweise die Reaktion von Phenol mit Phosgen zum Chloroformiat schnell, aber die Reaktion des Chloroformiats mit Phenol zu DPC langsamer abläuft, kann durch eine Verweilstrecke im zweiten Reaktionsraum in Strömungsrichtung nach der Phosgensynthese (so dass kein zusätzliches Phosgen in den zweiten Reaktionsraum übergeht) Phosgenvernichtung erfolgen.

In einer weiteren Ausführungsform des erfindungsgemäßen Reaktors umfasst der Reaktor eine Mehrzahl von ersten Reaktionsräumen, zweiten Reaktionsräumen und porösen Kohlenstoff-Membranen, wobei jeweils ein erster und zweiter Reaktionsraum durch eine poröse Kohlenstoff-Membran voneinander getrennt sind. So können flache, mehrschichtige und modulare Membranreaktoren erhalten werden.

In einer weiteren Ausführungsform des erfindungsgemäßen Reaktors ist der Reaktor zylindrisch aufgebaut mit von Innen nach Außen konzentrisch angeordnetem ersten Reaktionsraum und zweiten Reaktionsraum, wobei der erste und der zweite Reaktionsraum durch die poröse Kohlenstoff-Membran voneinander getrennt sind. Dann verhält sich der Reaktor im Prinzip wie ein Blasensäulenreaktor. Vorzugsweise sind mehrere dieser Reaktoren zu einem Rohrbündelreaktor zusammengefasst.

Der einzelne zylindrische Reaktor kann die folgenden Eigenschaften unabhängig voneinander aufweisen:
Durchmesser des zweiten Reaktionsraums: ≥ 3 bis ≤ 10 cm
Länge des zweiten Reaktionsraums: ≥ 3 bis ≤ 20 m

In einer weiteren Ausführungsform des erfindungsgemäßen Reaktors weisen der erste Reaktionsraum und/oder der zweite Reaktionsraum eine Querschnittsfläche rechtwinklig zur Strömungsrichtung des durchströmenden Fluids von ≥ 8 · 10⁻⁵ bis ≤ 8 · 10⁻⁴ m² auf.

In einer weiteren Ausführungsform des erfindungsgemäßen Reaktors umfasst der Reaktor eine Mehrzahl von ersten Reaktionsräumen, die von einem gemeinsamen zweiten Reaktionsraum umgeben sind.

Die vorliegende Erfindung wird anhand der nachfolgenden Figuren näher erläutert, ohne jedoch darauf beschränkt zu sein. Es zeigen:
- FIG. 1: einen Querschnitt durch einen Reaktor für das erfindungsgemäße Verfahren
- FIG. 2: einen Querschnitt durch einen weiteren Reaktor für das erfindungsgemäße Verfahren
- FIG. 3: einen Querschnitt durch einen weiteren Reaktor für das erfindungsgemäße Verfahren
- FIG. 4: einen Querschnitt durch einen weiteren Reaktor für das erfindungsgemäße Verfahren
- FIG. 5: einen Querschnitt durch einen weiteren Reaktor für das erfindungsgemäße Verfahren
- FIG. 6: einen Querschnitt durch einen weiteren Reaktor für das erfindungsgemäße Verfahren
- FIG. 7: Simulationsergebnisse für ein erfindungsgemäßes Verfahren
- FIG. 8: einen Querschnitt durch einen weiteren Reaktor für das erfindungsgemäße Verfahren

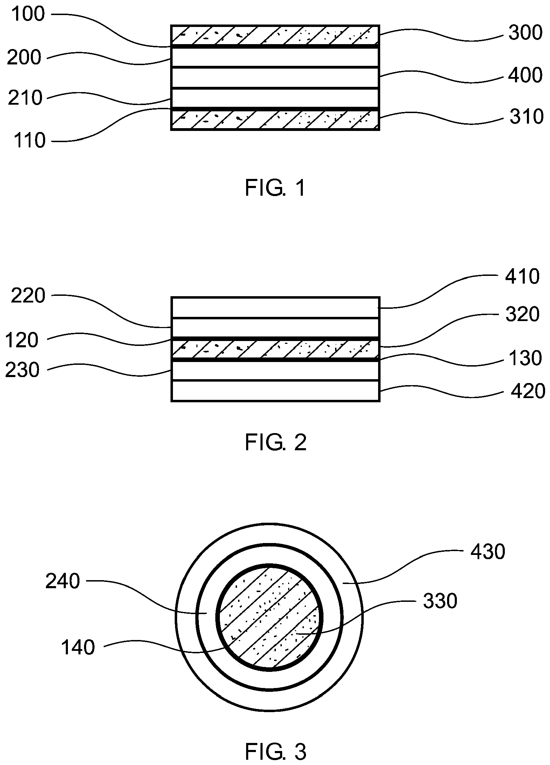

FIG. 1 zeigt einen schematischen Querschnitt durch einen Reaktor, wie er im erfindungsgemäßen Verfahren eingesetzt werden kann. Zwei poröse Kohlenstoff-Membranen 100, 110 trennen jeweils einen ersten Reaktionsraum 300, 310 von zweiten Reaktionsräumen 200, 210. Zentral angeordnet befindet sich ein weiterer Hohlraum 400, durch den ein Wärmeüberträgerfluid strömen kann, so dass der Hohlraum 400 die Funktion eines Wärmetauschers übernehmen kann. Die ersten Reaktionsräume 300, 310 beinhalten einen offenporigen Schaum, welcher neben einer stützenden Funktion auch eine bessere Gasdurchmischung bewirkt. Dieses kann zum Beispiel ein offenporiger Metallschaum sein. Kohlenmonoxid und Chlor werden in die ersten Reaktionsräume 300, 310 eingeleitet und reagieren unter Katalyse der Membranen 100, 110 zu Phosgen. Dieses Phosgen tritt durch die Poren der Membranen 100, 110 in die zweiten Reaktionsräume 200, 210 über. In den zweiten Reaktionsräumen 200, 210 liegt eine Hydroxyl-, Thiol-, Amino- und/oder Formamidgruppen enthaltende Verbindung wie Phenol vor, welche mit dem Phosgen reagiert. Zur Unterstützung dieser Reaktion kann ein Katalysator eingesetzt werden. Dieser kann als homogener Katalysator in zweiten Reaktionsräumen 200, 210 vorliegen. Alternativ oder zusätzlich kann ein heterogener Katalysator an der den zweiten Reaktionsräumen 200, 210 zugewandten Seite der Membranen 100, 110 vorliegen.

FIG. 2 zeigt einen schematischen Querschnitt durch einen weiteren Reaktor, wie er im erfindungsgemäßen Verfahren eingesetzt werden kann. Der hier dargestellte Reaktor unterscheidet sich vom Reaktor gemäß FIG. 1 durch die zentrale Anordnung des ersten Reaktionsraums 320, welcher nach oben und unten durch poröse Kohlenstoff-Membranen 120, 130 gegenüber zweiten Reaktionsräumen 220, 230 abgegrenzt ist. An den zweiten Reaktionsräumen 220, 230 angrenzend sind Hohlräume 410, 420 zur Aufnahme eines Wärmeüberträgerfluids angeordnet. Der in FIG. 2 gezeigte Reaktor ist vorteilhaft, wenn eine gegenüber dem Reaktor aus FIG. 1 eine größere Reaktionswärme abgeführt werden muss.

FIG. 3 zeigt einen schematischen Querschnitt durch einen weiteren Reaktor, wie er im erfindungsgemäßen Verfahren eingesetzt werden kann. Der Reaktor ist konzentrisch ausgeführt, so dass ein Rohrreaktor oder Rohrbündelreaktor realisiert werden kann. Die hier gezeigte Ansicht ist ein Querschnitt rechtwinklig zur Hauptachse des Reaktors. Im Inneren befindet sich der erste Reaktionsraum 330 mit einem offenporigen Schaum wie zuvor bereits beschrieben. Die poröse Kohlenstoff-Membran 140 trennt den ersten Reaktionsraum 330 vom zweiten Reaktionsraum 240. Hohlraum 430 dient wieder zur Aufnahme eines Wärmeüberträgerfluids.

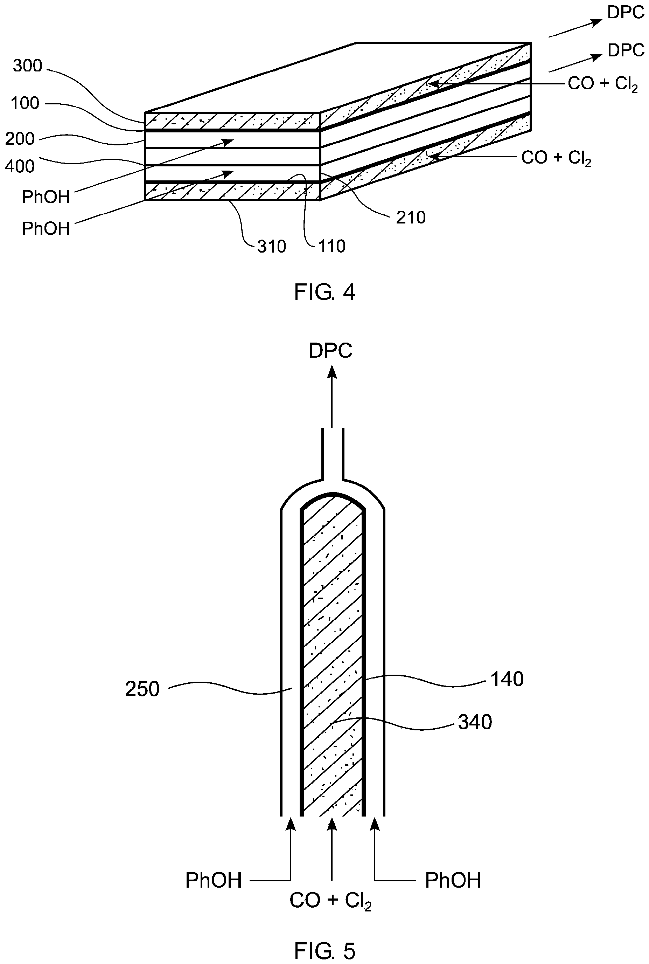

FIG. 4 zeigt einen schematischen Querschnitt durch einen weiteren Reaktor, wie er im erfindungsgemäßen Verfahren eingesetzt werden kann. Der Reaktor ist wie in FIG. 1 beschrieben. Hier soll beispielhaft die DPC-Synthese erläutert werden. CO- und Cl₂-Gas werden in die ersten Reaktionsräume 300, 310 eingeleitet und bilden bei Durchtritt durch die katalytisch aktive Kohlenstoff-Membran 100, 110 Phosgen. Nach Eintritt in die zweiten Reaktionsräume 200, 210 reagiert das in der Membran 100, 110 gebildete Phosgen mit Phenol (PhOH) über die Chloroformiat-Zwischenstufe zu Diphenylcarbonat (DPC). Die Stoffströme von Phenol sowie CO und Cl₂ verlaufen orthogonal zueinander. Zweckmäßigerweise strömt durch den Hohlraum 400 ein Wärmeüberträgerfluid ebenfalls orthogonal zur Fließrichtung des Phenols sowie entgegengesetzt zum CO und Cl₂-Strom. Dann lässt sich ein Kreuzstromwärmetauscher realisieren.

FIG. 5 zeigt einen schematischen Querschnitt durch einen weiteren Reaktor, wie er im erfindungsgemäßen Verfahren eingesetzt werden kann. Es handelt sich um einen Rohrreaktor, welcher ebenfalls Teil eines Rohrbündelreaktors sein kann. CO- und Cl₂-Gas werden in den ersten Reaktionsraum 340 eingeleitet und reagieren bei Durchtritt durch die katalytisch aktive, porösen Kohlenstoff-Membran 140 zu Phosgen. Bei Eintritt des Phosgens in den zweiten Reaktionsraum 250 reagiert es beispielsweise mit Phenol zu Diphenylcarbonat, unter intermediärer Bildung der Chloroformiat Zwischenstufe. Das Reaktionsprodukt verlässt den Rohrreaktor am oberen Ende. Im Fall der Rohr- oder Rohrbündelreaktoren kann durch ein frei fließendes Wärmeüberträgermedium direkt von außen gekühlt werden, so dass ein gesonderter Hohlraum wie in den zuvor geschilderten Reaktoren entbehrlich ist.

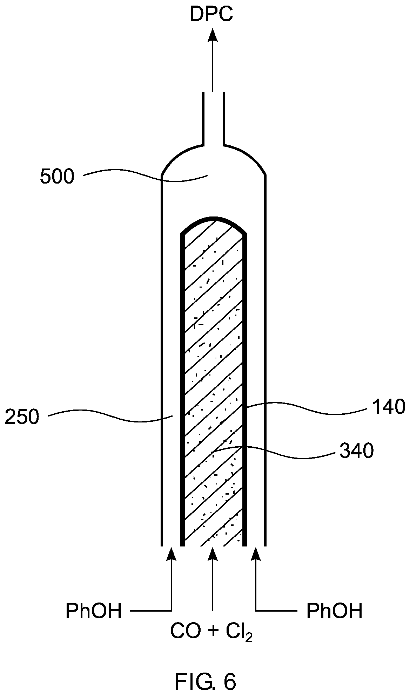

In der in FIG. 6 gezeigten Anordnung ist der Unterschied zum Reaktor gemäß FIG. 5, dass im zweiten Reaktionsraum 250 in Strömungsrichtung des Phenols gesehen nach der porösen Kohlenstoff-Membran 150 eine Verweilstrecke 500 vorliegt, in der die im zweiten Reaktionsraum 250 ablaufende Reaktion weiter fortschreiten kann. So kann bei Bedarf der Gesamtumsatz der Reaktion noch erhöht werden.

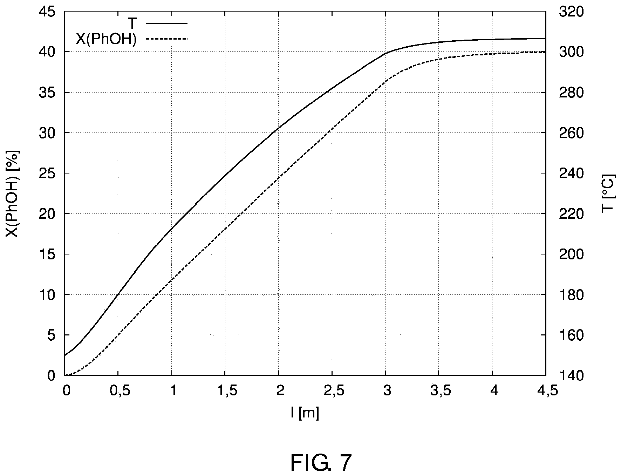

FIG. 7 zeigt Simulationsergebnisse für ein erfindungsgemäßes Verfahren. Modelliert wurde die Synthese von DPC durch Phosgenierung von Phenol auf Basis bekannter kinetischer Informationen. Kinetiken für homogene und heterogene Katalysen wurden aus internen Ergebnissen in das Modell eingebracht. Die physikalischen Stoffeigenschaften wurden dem Aspen Properties® Softwarepaket entnommen und wo möglich mit der experimentellen Detherm-Datenbank verglichen. Angewandte Spezifikationen waren: 99.9% Umsatz von Cl₂ in der Phosgensynthese, 100% Umsatz des Phosgens in der Phosgenierung von Phenol (kein Phosgen am Reaktorauslass), 300 °C Maximaltemperatur an der Membran. Als Druck wurden 25 bar verwendet, um Phosgen effektiv im flüssigen Phenol zu lösen und daher dessen Lebensdauer signifikant zu verringern. Das benötigte Molverhältnis von Phenol zu Phosgen für 100% Phosgenumsatz und zur Kühlung des Reaktors war >4:1. Der für die Modellierung verwendete Reaktor entspricht dem in FIG. 6 gezeigten Aufbau und besaß daher eine Verweilstrecke 500. In FIG. 7 sind der Phenolumsatz X(PhOH) und die Temperatur an der porösen Kohlenstoffmembran T gegen die Länge des Rohrreaktors aufgetragen. Der Reaktor hatte eine Gesamtlänge von 4,5 Metern. Der Teilabschnitt ab 3 Metern entspricht der Verweilstrecke für vollständigen Phosgenumsatz; die eigentliche Phosgensynthese erfolgt in den ersten 3 Metern des Reaktors. Die Starttemperatur des Phenols betrug 140 °C.

Eine Jahresproduktion von DPC von ca. 20000 Tonnen kann gemäß der obigen Modellrechnung in einem Rohrbündelreaktor mit ca. 400 Reaktoren gemäß FIG. 6 realisiert werden.

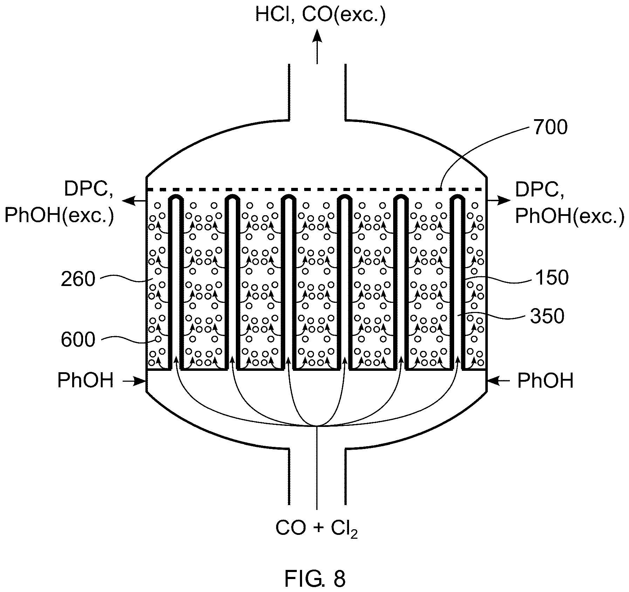

FIG. 8 zeigt einen schematischen Querschnitt durch einen weiteren Reaktor für das erfindungsgemäße Verfahren. Wie zu erkennen ist, liegt eine Mehrzahl von einseitig offenen ersten Reaktionsräumen 350 vor, welche durch Membranen 150 von einem gemeinsamen zweiten Reaktionsraum 260 getrennt sind. Am unteren Ende des Reaktors werden CO und Chlorgas eingebracht. Die Gasmischung gelangt in die ersten Reaktionsräume und reagiert unter Katalyse der Membranen zu Phosgen, welches durch die Membranen hindurchtritt. Dieses ist schematisch durch Pfeile und die Gasbläschen 600 dargestellt. Am unteren Ende des zweiten Reaktionsraumes wird Phenol eingetragen. Dieses liegt in flüssiger Phase vor, beispielsweise geschmolzen oder in Lösung. Die Oberfläche der flüssigen Phase im zweiten Reaktionsraum wird durch die gestrichelte Linie 700 dargestellt. Dementsprechend befindet sich oberhalb der flüssigen Phase eine Gasphase. Im zweiten Reaktionsraum reagiert das eingetragene Phenol mit dem durch die Membranen hindurchgetretenen Phosgen zu DPC. Die Produktmischung aus DPC und nicht umgesetztem Phenol ("PhOH(exc.)") wird am oberen Ende des zweiten Reaktionsraums entnommen. Am oberen Ende des Reaktors werden als gasförmiges Produkt HCl sowie nicht umgesetztes CO ("CO(exc.)") herausgeführt.

Eine Jahresproduktion von DPC von ca. 20000 Tonnen kann gemäß der zuvor erwähnten Modellrechnung in einem Reaktor mit ca. 400 ersten Reaktionsräumen gemäß FIG. 8 realisiert werden.

## Patentansprüche

1. Verfahren zur Reaktion einer ersten Verbindung mit einer zweiten Verbindung,
wobei die erste Verbindung eine Gefahrstoffkennzeichnung gemäß GHS von GHS06 aufweist und aus der Reaktion von mindestens einer ersten fluiden Vorläuferverbindung und einer zweiten fluiden Vorläuferverbindung erhältlich ist
und wobei die zweite Verbindung mit der ersten Verbindung zu einer chemischen Reaktion in der Lage ist,
umfassend die Schritte:
(I) Bereitstellen eines Reaktors, welcher einen ersten Reaktionsraum (300, 310, 320, 330, 340, 350) und einen zweiten Reaktionsraum (200, 210, 220, 230, 240, 250, 260) umfasst, wobei der erste und der zweite Reaktionsraum durch eine poröse Kohlenstoff-Membran (100, 110, 120, 130, 140, 150) voneinander getrennt sind;
(II) Bereitstellen der ersten und der zweiten Vorläuferverbindung im ersten Reaktionsraum;
sowie gleichzeitig
(III) Bereitstellen der zweiten Verbindung in dem zweiten Reaktionsraum;
wobei die poröse Kohlenstoff-Membran eingerichtet ist, um:
- die Reaktion der ersten und zweiten Vorläuferverbindung zur ersten Verbindung zu katalysieren und
- die gebildete erste Verbindung in den zweiten Reaktionsraum übergehen zu lassen.

2. Verfahren gemäß Anspruch 1, wobei die erste Verbindung Phosgen ist, die erste Vorläuferverbindung Kohlenmonoxid ist, die zweite Vorläuferverbindung Chlor ist und die zweite Verbindung eine Hydroxyl-, Thiol-, Amino- und/oder Formamidgruppen enthaltende Verbindung ist.

3. Verfahren gemäß Anspruch 1 oder 2, wobei die poröse Kohlenstoff-Membran (100, 110, 120, 130, 140, 150) eine nominale Porengröße, bestimmt mittels Quecksilber-Porosimetrie (ISO 15901-1), von ≥ 0,01 bis ≤ 10 µm aufweist.

4. Verfahren gemäß einem der Ansprüche 1 bis 3, wobei die poröse Kohlenstoff-Membran (100, 110, 120, 130, 140, 150) weiterhin einen Katalysator für die Reaktion der ersten Verbindung mit der zweiten Verbindung umfasst, welcher zumindest teilweise auf der dem zweiten Reaktionsraum (200, 210, 220, 230, 240, 250, 260) zugewandten Seite der porösen Kohlenstoff-Membran angeordnet ist.

5. Verfahren gemäß einem der Ansprüche 1 bis 4, wobei im zweiten Reaktionsraum (200, 210, 220, 230, 240, 250, 260) weiterhin ein homogener Katalysator vorliegt.

6. Verfahren gemäß einem der Ansprüche 1 bis 5, wobei im ersten Reaktionsraum (300, 310, 320, 330, 340, 350) weiterhin ein offenzelliger Schaum vorliegt.

7. Verfahren gemäß einem der Ansprüche 1 bis 6, wobei der Reaktor weiterhin einen Hohlraum (400, 410, 420, 430) zur Aufnahme eines Wärmeüberträgerfluids umfasst.

8. Verfahren gemäß einem der Ansprüche 1 bis 7, wobei der Reaktor weiterhin eine Verweilstrecke (500) zur Vervollständigung der Reaktion der ersten Verbindung mit der zweiten Verbindung umfasst.

9. Verfahren gemäß einem der Ansprüche 1 bis 8, wobei der Reaktor eine Mehrzahl von ersten Reaktionsräumen (300, 310, 320, 330, 340, 350), zweiten Reaktionsräumen (200, 210, 220, 230, 240, 250, 260) und porösen Kohlenstoff-Membranen (100, 110, 120, 130, 140, 150) umfasst, wobei jeweils ein erster und zweiter Reaktionsraum durch eine poröse Kohlenstoff-Membran voneinander getrennt sind.

10. Verfahren gemäß einem der Ansprüche 1 bis 8, wobei der Reaktor zylindrisch aufgebaut ist mit von Innen nach Außen konzentrisch angeordnetem ersten Reaktionsraum (300, 310, 320, 330, 340, 350) und zweiten Reaktionsraum (200, 210, 220, 230, 240, 250, 260), wobei der erste und der zweite Reaktionsraum durch die poröse Kohlenstoff-Membran (100, 110, 120, 130, 140, 150) voneinander getrennt sind.

11. Verfahren gemäß einem der Ansprüche 1 bis 10, wobei der erste Reaktionsraum (300, 310, 320, 330, 340, 350) und/oder der zweite Reaktionsraum (200, 210, 220, 230, 240, 250, 260) eine Querschnittsfläche rechtwinklig zur Strömungsrichtung des durchströmenden Fluids von ≥ 8 · 10⁻⁵ bis ≤ 8 · 10⁻⁴ m² aufweisen.

12. Verfahren gemäß einem der Ansprüche 1 bis 8 oder 11, wobei der Reaktor eine Mehrzahl von ersten Reaktionsräumen (300, 310, 320, 330, 340, 350) umfasst, die von einem gemeinsamen zweiten Reaktionsraum (200, 210, 220, 230, 240, 250, 260) umgeben sind.

13. Reaktor zur Reaktion von Phosgen mit Hydroxyl-, Thiol-, Amino- und/oder Formamidgruppen enthaltenden Verbindungen, umfassend:
- einen ersten Reaktionsraum (300, 310, 320, 330, 340, 350) und einen zweiten Reaktionsraum (200, 210, 220, 230, 240, 250, 260), wobei der erste und der zweite Reaktionsraum durch eine poröse Kohlenstoff-Membran (100, 110, 120, 130, 140, 150) voneinander getrennt sind;
und
- einen Katalysator für die Reaktion von Phosgen mit der Hydroxyl-, Thiol-, Amino- und/oder Formamidgruppen enthaltenden Verbindung, welcher zumindest teilweise auf der dem zweiten Reaktionsraum zugewandten Seite der porösen Kohlenstoff-Membran angeordnet ist.

14. Reaktor gemäß Anspruch 13, wobei im ersten Reaktionsraum (300, 310, 320, 330, 340, 350) weiterhin ein offenzelliger Schaum vorliegt.

15. Reaktor gemäß Anspruch 13 oder 14, wobei der Reaktor eine Mehrzahl von ersten Reaktionsräumen (300, 310, 320, 330, 340, 350) umfasst, die von einem gemeinsamen zweiten Reaktionsraum (200, 210, 220, 230, 240, 250, 260) umgeben sind.

## Claims

1. Method of reacting a first compound with a second compound,
wherein the first compound has a GHS hazard identification of GHS06 and is obtainable from the reaction of at least one first fluid precursor compound and a second fluid precursor compound
and wherein the second compound is capable of a chemical reaction with the first compound,
comprising the steps of:
(I) providing a reactor comprising a first reaction space (300, 310, 320, 330, 340, 350) and a second reaction space (200, 210, 220, 230, 240, 250, 260), wherein the first and second reaction spaces are separated from one another by a porous carbon membrane (100, 110, 120, 130, 140, 150);
(II) providing the first and second precursor compounds in the first reaction space;
and simultaneously
(III) providing the second compound in the second reaction space;
wherein the porous carbon membrane is set up to:
- catalyze the reaction of the first and second precursor compounds to give the first compound and
- allow the first compound formed to move into the second reaction space.

2. Method according to Claim 1, wherein the first compound is phosgene, the first precursor compound is carbon monoxide, the second precursor compound is chlorine and the second compound is a compound containing hydroxyl, thiol, amino and/or formamide groups.

3. Method according to Claim 1 or 2, wherein the porous carbon membrane (100, 110, 120, 130, 140, 150) has a nominal pore size, determined by means of mercury porosimetry (ISO 15901-1), of ≥ 0.01 to ≤ 10 µm.

4. Method according to any of Claims 1 to 3, wherein the porous carbon membrane (100, 110, 120, 130, 140, 150) further comprises a catalyst for the reaction of the first compound with the second compound, arranged at least partly on the side of the porous carbon membrane facing the second reaction space (200, 210, 220, 230, 240, 250, 260).

5. Method according to any of Claims 1 to 4, wherein a homogeneous catalyst is additionally present in the second reaction space (200, 210, 220, 230, 240, 250, 260).

6. Method according to any of Claims 1 to 5, wherein an open-cell foam is additionally present in the first reaction space (300, 310, 320, 330, 340, 350).

7. Method according to any of Claims 1 to 6, wherein the reactor further comprises a cavity (400, 410, 420, 430) to accommodate a heat transfer fluid.

8. Method according to any of Claims 1 to 7, wherein the reactor additionally comprises a dwell zone (500) to complete the reaction of the first compound with the second compound.

9. Method according to any of Claims 1 to 8, wherein the reactor comprises a multitude of first reaction spaces (300, 310, 320, 330, 340, 350), second reaction spaces (200, 210, 220, 230, 240, 250, 260) and porous carbon membranes (100, 110, 120, 130, 140, 150), wherein one first and one second reaction space are separated from one another in each case by a porous carbon membrane.

10. Method according to any of Claims 1 to 8, wherein the reactor has a cylindrical construction with first reaction space (300, 310, 320, 330, 340, 350) and second reaction space (200, 210, 220, 230, 240, 250, 260) arranged concentrically from the inside outward, wherein the first and second reaction spaces are separated from one another by the porous carbon membrane (100, 110, 120, 130, 140, 150).

11. Method according to any of Claims 1 to 10, wherein the first reaction space (300, 310, 320, 330, 340, 350) and/or the second reaction space (200, 210, 220, 230, 240, 250, 260) have a cross-sectional area at right angles to the flow direction of the fluid flowing through of ≥ 8 · 10⁻⁵ to ≤ 8 · 10⁻⁴ m²·

12. Method according to any of Claims 1 to 8 or 11, wherein the reactor comprises a multitude of first reaction spaces (300, 310, 320, 330, 340, 350) surrounded by a common second reaction space (200, 210, 220, 230, 240, 250, 260).

13. A reactor for reaction of phosgene with compounds containing hydroxyl, thiol, amino and/or formamide groups, comprising:
- a first reaction space (300, 310, 320, 330, 340, 350) and a second reaction space (200, 210, 220, 230, 240, 250, 260), wherein the first and second reaction spaces are separated from one another by a porous carbon membrane (100, 110, 120, 130, 140, 150);
and
- a catalyst for the reaction of phosgene with the compound containing hydroxyl, thiol, amino and/or formamide groups, arranged at least partly on the side of the porous carbon membrane facing the second reaction space.

14. Reactor according to Claim 13, wherein an open-cell foam is additionally present in the first reaction space (300, 310, 320, 330, 340, 350).

15. Reactor according to Claim 13 or 14, wherein the reactor comprises a multitude of first reaction spaces (300, 310, 320, 330, 340, 350) surrounded by a common second reaction space (200, 210, 220, 230, 240, 250, 260).

## Revendications

1. Procédé de réaction d'un premier composé avec un deuxième composé,
le premier composé présentant une classe de danger selon le SGH de SGH06, et pouvant être obtenu par la réaction d'au moins un premier composé précurseur fluide et d'un deuxième composé précurseur fluide,
et le deuxième composé étant apte à une réaction chimique avec le premier composé,
comprenant les étapes suivantes :
(I) la préparation d'un réacteur, qui comprend une première chambre de réaction (300, 310, 320, 330, 340, 350) et une deuxième chambre de réaction (200, 210, 220, 230, 240, 250, 260), la première et la deuxième chambre de réaction étant séparées l'une de l'autre par une membrane poreuse en carbone (100, 110, 120, 130, 140, 150) ;
(II) la préparation du premier et du deuxième composé précurseur dans la première chambre de réaction ;
et simultanément
(III) la préparation du deuxième composé dans la deuxième chambre de réaction ;
la membrane poreuse en carbone étant conçue pour :
- catalyser la réaction du premier et du deuxième composé précurseur pour former le premier composé, et
- laisser passer le premier composé formé dans la deuxième chambre de réaction.

2. Procédé selon la revendication 1, dans lequel le premier composé est le phosgène, le premier composé précurseur est le monoxyde de carbone, le deuxième composé précurseur est le chlore et le deuxième composé est un composé contenant des groupes hydroxyle, thiol, amino et/ou formamide.

3. Procédé selon la revendication 1 ou 2, dans lequel la membrane poreuse en carbone (100, 110, 120, 130, 140, 150) présente une taille de pores nominale, déterminée par porosimétrie au mercure (ISO 15901-1), de ≥ 0,01 à ≤ 10 µm.

4. Procédé selon l'une quelconque des revendications 1 à 3, dans lequel la membrane poreuse en carbone (100, 110, 120, 130, 140, 150) comprend en outre un catalyseur pour la réaction du premier composé avec le deuxième composé, qui est agencé au moins en partie sur le côté orienté vers la deuxième chambre de réaction (200, 210, 220, 230, 240, 250, 260) de la membrane poreuse en carbone.

5. Procédé selon l'une quelconque des revendications 1 à 4, dans lequel un catalyseur homogène est en outre présent dans la deuxième chambre de réaction (200, 210, 220, 230, 240, 250, 260).

6. Procédé selon l'une quelconque des revendications 1 à 5, dans lequel une mousse à cellules ouvertes est en outre présente dans la première chambre de réaction (300, 310, 320, 330, 340, 350).

7. Procédé selon l'une quelconque des revendications 1 à 6, dans lequel le réacteur comprend en outre une cavité (400, 410, 420, 430) destinée à recevoir un fluide de transfert de chaleur.

8. Procédé selon l'une quelconque des revendications 1 à 7, dans lequel le réacteur comprend en outre une section de séjour (500) pour la complétion de la réaction du premier composé avec le deuxième composé.

9. Procédé selon l'une quelconque des revendications 1 à 8, dans lequel le réacteur comprend une pluralité de premières chambres de réaction (300, 310, 320, 330, 340, 350), de deuxièmes chambres de réaction (200, 210, 220, 230, 240, 250, 260) et de membranes poreuses en carbone (100, 110, 120, 130, 140, 150), une première et une deuxième chambre de réaction étant à chaque fois séparées l'une de l'autre par une membrane poreuse en carbone.

10. Procédé selon l'une quelconque des revendications 1 à 8, dans lequel le réacteur est configuré sous forme cylindrique avec des premières chambres de réaction (300, 310, 320, 330, 340, 350) et des deuxièmes chambres de réaction (200, 210, 220, 230, 240, 250, 260) agencées concentriquement de l'intérieur vers l'extérieur, la première et la deuxième chambre de réaction étant séparées l'une de l'autre par la membrane poreuse en carbone (100, 110, 120, 130, 140, 150) .

11. Procédé selon l'une quelconque des revendications 1 à 10, dans lequel la première chambre de réaction (300, 310, 320, 330, 340, 350) et/ou la deuxième chambre de réaction (200, 210, 220, 230, 240, 250, 260) présentent une surface de section transversale perpendiculairement à la direction d'écoulement du fluide qui les traverse de ≥ 8·10⁻⁵ à ≤ 8·10⁻⁴ m²·

12. Procédé selon l'une quelconque des revendications 1 à 8 ou 11, dans lequel le réacteur comprend une pluralité de premières chambres de réaction (300, 310, 320, 330, 340, 350), qui sont entourées par une deuxième chambre de réaction commune (200, 210, 220, 230, 240, 250, 260).

13. Réacteur pour la réaction de phosgène avec des composés contenant des groupes hydroxyle, thiol, amino et/ou formamide, comprenant :
- une première chambre de réaction (300, 310, 320, 330, 340, 350) et une deuxième chambre de réaction (200, 210, 220, 230, 240, 250, 260), la première et la deuxième chambre de réaction étant séparées l'une de l'autre par une membrane poreuse en carbone (100, 110, 120, 130, 140, 150) ;
et
- un catalyseur pour la réaction de phosgène avec le composé contenant des groupes hydroxyle, thiol, amino et/ou formamide, qui est agencé au moins en partie sur le côté orienté vers la deuxième chambre de réaction de la membrane poreuse en carbone.

14. Réacteur selon la revendication 13, dans lequel une mousse à cellules ouvertes est en outre présente dans la première chambre de réaction (300, 310, 320, 330, 340, 350).

15. Réacteur selon la revendication 13 ou 14, dans lequel le réacteur comprend une pluralité de premières chambres de réaction (300, 310, 320, 330, 340, 350), qui sont entourées par une deuxième chambre de réaction commune (200, 210, 220, 230, 240, 250, 260).
